# EUROPÄISCHE PATENTSCHRIFT

(11) **EP 1 870 133 B1**
(45) Veröffentlichungstag und Bekanntmachung des Hinweises auf die Patenterteilung: **15.10.2008**
(21) Anmeldenummer: 06012835.2
(22) Anmeldetag: 22.06.2006
(51) Int. Cl.: A61N 5/10, A61B 6/00, A61B 5/087

(54) **Vorrichtung zur räumlichen Verfolgung einer beweglichen Lage eines Köperteils**
Device for spatial tracking of a moving position of a body part
Appareil de suivi spatial d'une position mobile d'une zone corporelle

(43) Veröffentlichungstag der Anmeldung: 26.12.2007
(73) Patentinhaber: Siemens Schweiz AG, 8047 Zürich (CH)
(72) Erfinder: Broennimann, Thomas, 4704 Wolfisberg (CH)
(74) Vertreter: Fischer, Michael

(56) Entgegenhaltungen:
- EP-A- 1 369 085
- WO-A-98/52635
- DE-A1- 10 310 127
- US-A- 6 118 847
- US-A1- 2004 254 492
- US-B1- 6 298 260
- US-B1- 6 501 981

## Beschreibung

Die Erfindung betrifft eine Vorrichtung zur räumlichen Verfolgung einer beweglichen Lage eines Körperteils nach dem Oberbegriff des Anspruches 1.

Präzisere radioonkologische Planung von Bestrahlungsvolumen im Thoraxbereich sind heutzutage ein wichtiges Thema, da ihre Zuverlässigkeit für eine erfolgreiche Behandlung noch nicht gesichert ist. Insbesondere, beim Ein- und Ausatmen eines auf einer geeigneten Fläche liegenden Patienten mit einem zu bestrahlenden Tumor (oder Karzynom, etc) im Thorax-Bereich ändert der Tumor oder Karzinom seine topographische Lage im Thorax, hauptsächlich durch den Atem des Patienten. In anderen Worten ist bei der Strahlentherapie eine adaptive räumliche Verfolgung des beweglichen Tumors erforderlich, damit lediglich den gesamten Tumor und möglichst keine anderen, dem Tumor benachbarten Organen oder gesunden Zellen abgestrahlt werden. Damit dieses Artefakt während der tatsächlichen Behandlung berücksichtigt wird, wird z.B. mittels regelmässiger Computertomographien (CT) während einer der/den Bestrahlungsphase(n) vorgängigen Phase eine Planung bzw. eine Kalibrierung eines Volumen im Thorax durchgeführt, in welchem sich der Tumor in Abhängigkeit der Atmungszyklen bewegt. Anschliessend wird zumindest ein als Referenzlage bezeichneter Teil dieses ermittelten Volumen gewählt, für welchen die Strahlungsenergie konzentriert bzw. fokussiert wird. D.h. wenn es bei der Bestrahlungsphase gerechnet wird, dass der bewegliche Tumor in der Referenzlage sich befindet, wird die Bestrahlungsvorrichtung eingeschaltet. Andersfall wird die Bestrahlungsvorrichtung ausgeschaltet, so dass keine anderen Organen oder gesunden Zellen als den Tumor bestrahlt werden bzw, hohe Schaden erfahren. Dabei ist es auch einfach zu begreifen, dass eine räumliche Unsicherheit immer vorliegt, da zwischen der Kalibrierung bei der Computertomographie und der tatsächlichen Bestrahlungsphase der Patient und dessen Atmungseigenschaften sich ändern können. Damit besteht die Gefahr, dass das gezielte Volumen bzw. die Referenzlage bei der Strahlentherapie teilweise oder gar nicht mehr von Gebrauch sind. zur Minimierung der Unsicherheit werden meistens überwachungsmitteln des Thorax eingesetzt, mit welchen eine bessere Abschätzung der Position des Tumors hergeleitet wird. Leider kann diese Abschätzung nicht durch eine während der Bestrahlungsphase permanente und direkte Messung immer erfolgen, da dies nur mit einer weiteren Bestrahlung (Röntgen, Ionisierungstechnik, Tomographie, etc) für den bereits schwachen und hoch belasteten Patient verbunden wäre. Deshalb werden indirekte Mitteln Überwachungsmitteln der Geometrie des durch vorberechnete Atmungseigenschaften verformbaren Thorax eingesetzt, und anschliessend aus rückgewonnenen Daten der variablen Geometrie des Thorax wird eine Lage des Tumors mit einem hochleistenden Computer errechnet. Es sind dabei viele Verfahren zur Ermittlung der Geometrie des Thorax und ferner zur Berechnung der Lage des Tumors möglich: optische Methoden mit Kameras, optische Sensoren wie Lichtschränke, Markierungen am Patient für stereo-photogrammetrische Verfahren, dreidimensionalen, möglichst dynamischen Abtastverfahren des Brustkorbs, etc. Noch mal sind es hier alle indirekte und oft sehr aufwendige Verfahren, welche die Lage des Tumors lediglich mit einer zu hohen Unsicherheit abschätzen können. Eine weitere Methode zur räumlichen Verfolgung des Tumors im Thorax besteht darin, einen elastischen Brustgurt an dem Patient einzusetzen, welche einen oder mehrere Drucksensoren zur Ermittlung der Verformung des Thorax während der Atmung des Patienten aufweist. Der Brustgurt wird sowohl bei der Kalibrierung (Computertomographie) als bei der tatsächlichen Strahlentherapie verwendet, so dass gemäß der Verformung des Thorax eine räumliche Verfolgung vereinfacht wird. Aufgrund des periodischen manuellen Einziehens des Brutgurts am Patienten und aufgrund seiner möglichen körperlichen Veränderungen (z.B. durch Gewicht- und/oder Muskelabnahme oder durch andere Lungen bezogenen Atemeigenschaften) kann die Positionierung des Brustgurts an zumindest der gleichen Thoraxstelle bzw. die Messung der Verformung des Thorax mittels des Brustgurts ungenau sein. Daher mangelt diese Methode an Reproduzierbarkeit. Bei einer möglichen Brustamputierung an einer Patientin besteht weiterhin der Nachteil, dass der Brustgurt impliziterweise sehr ungeeignet bleibt.

Weitere Verfahren mit aktiver Mithilfe des Patienten sind auch heutzutage verwendet. Z.B. wird der Patient ganz einatmen und anschließend die Luft anhalten, so dass während dieser letzten Phase (der Tumor liegt hier an einer festen Stelle, welche Ziel der Strahlung ist) er das Auslösen der Bestrahlung steuern kann. Hier können solche Verfahren eine schwere Herausforderung für den Patient sein, bzw. von menschlichen Fehlern leiden.

Zusammengefasst weisen viele Methoden zur räumlichen Verfolgung des Tumors mittels einer Erfassung von Profiländerungen des Thorax Ungenauigkeit, geringe Reproduzierbarkeit, eine zu hohe Belastung gegenüber dem schwerkranken Patienten auf, oder sind mindestens sehr aufwendig und teuer, wenn die Genauigkeit erzielt sein soll.

Aus DE 103 10 127 A1 ist eine Vorrichtung zur besseren Abschätzung der Lage eines Tumors im Körper bekannt, bei welcher ferner ein Beschleuniger zur Strahlung des Tumors in Verbindung mit der atembedingten Bewegung des Tumors über Minima und Maxima von ermittelten Atemwerten getriggert wird. Zur Ermittlung der Atemwerte wird ein Ausführungsbeispiel beschrieben, bei welchem eine Nasen-Mundmaske mit einem Drucksensor an dem Patient eingesetzt wird. Weitere Details in Bezug auf dem Drucksensor und der räumlichen Verfolgung des Tumors sind allerdings nicht angegeben.

Der Erfindung liegt daher die Aufgabe zugrunde, eine Methode bzw. eine einfache Vorrichtung zur zuverlässigen räumlichen Verfolgung einer Lage (z.B. eines Tumors) im Thoraxbereich anzugeben, welche eine kostengünstige und sterilisationsfreie Nasen-Mundmaske mit einem integrierten Drucksensor verwendet. Eine weitere Aufgabe ist es, im Falle der Strahlentherapie die Sicherheit und den Komfort zur Heilung des Patienten effektiv zu steigern, indem die räumliche Verfolgung eines Tumors präziser, schneller und sowieso ohne aktivem bzw. "markantem" Einsatz des Patienten erfolgt.

Erfindungsgemäß wird die Aufgabe durch die Merkmale des Anspruches 1 gelöst.

Dabei wird eine Vorrichtung zur räumlichen Verfolgung einer beweglichen Lage (wie eines Tumors) eines Körperteils im verformbaren Thorax-Bereich eines atmenden Menschen vorgeschlagen. Diese Vorrichtung weist eine Messeinheit (wie eine Nasen-Mund-Maske) zur Ermittlung eines vom Kopf des Menschen ein- und ausgehenden Luftstromes auf, welche mit einem Auswertungsmodul derart gekoppelt ist, dass ein aus der Messeinheit ausgehendes Meßsignal ein Maß für zumindest eine im Auswertungsmodul ermittelbare Entfernung zwischen der beweglichen Lage des Körperteils und einer Referenzlage (z.B. des Energiefokussen der Bestrahlungseinrichtung zur Bestrahlung des Tumors) bildet.

Im folgendem wird es aus Klarheitsgründen angenommen, dass eine Nasen-Mundmaske verwendet wird, jedoch könnten weitere Methoden zur Ermittlung eines vom Kopf des Menschen ein- und ausgehenden Luftstromes vorstellbar sein (z.B. durch eine Temperatur- oder eine druckbasierte Messung in der Nähe von Atemwegen des Patienten). Insbesondere ist es vorteilhaft, dass die Messeinheit eine Nasen-Mundmaske umfasst, über welche der ein- und ausgehenden Luftstrom als von Mund und Nase summiertes Atemluftstrom sehr genau- ermittelt werden kann. Es spielt damit keine Rolle, ob der Patient durch Mund oder Nase atmet. Die Maske ist am liebsten eine Einwegmaske aus leichtem Kunststoff und wird nach einer Bestrahlung entsorgt. Dabei entfällt daher auch eine notwendige Sterilisation. Dadurch ist ferner ein Brustgurt nicht mehr notwendig, so dass nun die Methode gegen Unsicherheit der Gurtposition, Messungsreproduzierbarkeit bzw. Einziehen an dem schwerkranken Patient(in) komplett geeignet ist. Die Methode ermöglicht ein passives Verhalten des Patienten, da keine aktive Mitwirkung von ihm verlangt wird.

Es können vorkalibrierte Messdaten der räumlichen Bewegung der Lage des Körperteils in Anhängigkeit von über die Nasen-Mundmaske ermittelte Atmungseigenschaften eines Patienten in einer hard- oder/und softwaremässige Planungsoberfläche erst mal (z.B. durch Computertomographie oder durch ein Abbildungsverfahren mit Magnetresonanz, etc) gespeichert werden, derart dass eine anschließende Steuerung einer ein- und ausschaltbaren Bestrahlungseinrichtung für den Patient zumindest simulierbar ist und ferner in Verbindung mit einem geeigneten Therapieplanungssystem steuerbar ist. Während aller dieser Phasen kann die Nasen-Mundmaske verwendet werden.

Mit Hilfe von so genannten "Elektrorespirographen" (zeitlich aufgenommene Verläufe des Atemdruckes) und anhand von aufgenommenen Schnittbildern aus einer Computertomographie (CT) gestützten Planung kann dadurch die Lage des Tumors in Abhängigkeit der Atmungszyklen bestimmt werden und anschliessend der Strahl eines Linear- oder Protonenbeschleunigers an einem festgelegten Punkt des Atmungszyklus getriggert werden. Sollte ferner die Bestrahlungseinrichtung ein (elektromagnetisches, mechanisches oder mit adaptiven Blenden gestattetes, etc) Umlenkmittel der Strahlung aufweisen, bildet das Messsignal aus der Messeinheit (Nasen-Mundmaske und Drucksensor) eine genaue Maß der Umlenkungsamplituden, so dass die Messeinheit die Umlenkung des Strahls zur räumlichen Verfolgung des Tumors direkt steuern/triggern kann. Ist es nicht der Fall, d.h. die Strahlen der Bestrahlungseinrichtung festgelegt sind) schaltet sich die Einrichtung ein, nur wenn sich der Tumor innerhalb der tolerierten Referenzlage befindet. Damit eignet sich die erfindungsgemässe Vorrichtung für alle Arten von Bestrahlungseinrichtung, d.h. mit oder ohne Umlenkmitteln der Strahlungsziele.

Vorteilhafte Ausgestaltungen der Erfindung sind in den Unteransprüchen dargelegt.

Anschließend wird die Erfindung in einem Ausführungsbeispiel anhand der Zeichnungen erläutert.

### Dabei zeigen

- FIG 1: eine erfindungsgemäße Vorrichtung zur räumlichen Verfolgung eines Tumors,
- FIG 2: Randbedingungen der Bestrahlung in einem Atemzyklus,
- FIG 3: Grundkomponente der erfindungsgemäßen Vorrichtung.

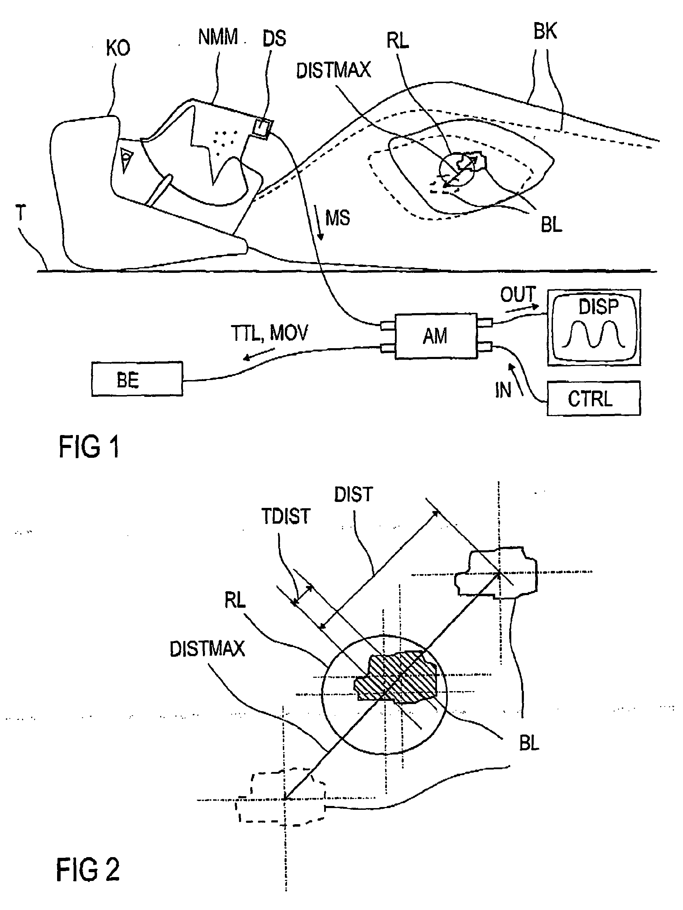

Aus FIG 1 ist eine erfindungsgemäße Vorrichtung zur räumlichen Verfolgung eines beweglichen Tumors BL im verformbaren Brustkorb BK eines auf einem Liegetisch T liegenden Patient dargestellt. Zwei Positionen für den Tumor BL (sowie den Brustkorb BK - siehe volle und gestrichene Zeichenelemente) sind angezeigt, für welche ein volles Ein- und Ausatmen erreicht wird. Daher weisen beide Positionen eine maximale Entfernung DISTMAX auf, innerhalb welcher eine bestimmte Referenzlage RL gemäß einer vorgängigen Strahlungsplanung (bzw. einem Therapieplanungssystem) gewählt werden kann, um eine dortige Strahlung zu konzentrieren, wenn sich der Tumor BL in der Referenzlage RL.befindet. Zum Kenntnis dieser Bedingung wird über eine Nasen-Mundmaske NMM am Kopf KO des Patienten und einen in der Maske integrierten Drucksensor DS, vorzugsweise mittels eines Piezo-resistiven Elements, oder einen anderen Sensor wie einen Dynamometer oder einen Temperatursensor ein Meßsignal MS erzeugt, welches die exakte Atezmaznplitude des Patienten an jeder Zeit abgibt. Da eine über eine Vorphase (Z.B. eine Computertomographie) bekannte Beziehung zwischen einer Atemamplitude und zumindest zwei unterschiedlichen Lagen BL des Tumors vorliegt, ist eine genaue räumliche Verfolgung des Tumors bzw. eine sichere Planung der topographisch zielorientierten Bestrahlung gewährleistet. Dabei ist die Messeinheit NMM zur Ermittlung eines vom Kopf des Menschen ein- und ausgehenden Luftstromes mit einem Auswertungsmodul AM derart gekoppelt ist, dass das aus der Messeinheit ausgehendes Meßsignal MS ein Maß für zumindest eine im Auswertungsmodul ermittelbare Entfernung zwischen der beweglichen Lage des Körperteils BL und der Referenzlage RL bildet. Das Auswertungsmodul AM weist hier als Eingangsignale das Meßsignal MS und ein weiteres Steuersignal TN aus einer Bedienereinheit CTRL (Rechner/Steuerungseinheit seitens des Krankenpflegers in Verbindung mit der Plattform für die Planungsoberfläche bei der Computertomographie sowie für die Strahlungstherapie) und als Ausgangsignale ein Monitorsignal OUT zur Anzeige der erfindungsgemässen Elektrorespirographen an einem Monitor DISP und ein Steuer- bzw. Triggersignal TTL zur Ein- und Ausschaltung der Bestrahlungseinrichtung BE auf.

Ein weiteres, aus dem Auswertungsmodul AM abgegebenes Steuersignal MOV kann ein Positionierungsmodul des Menschenkörpers und/oder der Bestrahlungseinrichtung BE steuern, so dass räumliche Abweichungen zwischen der beweglichen Lage des Körperteils und der Referenzlage minimiert werden. Eine erste Lösung besteht z.B. darin, den Liegetisch T gemäss dem A-temrhytmus hoch und runter zu fahren, jedoch muss die Amplitude dieses Verfahrens gering (cm-Bereich) bleiben, da der Patient ein Unwohlgefühl schnell erleben könnte. Vielmehr kann das Signal MOV als Steuersignal zur aktiven Umlenkung des Strahlengangs der Bestrahlungseinrichtung verwendet werden. Ziel dabei ist eine aktive, zyklische Neupositionierung des Tumors BL im Bereich der Referenzlage RL zu gewährleisten.

Es wird auch vorgesehen, dass bei unerwarteten, wie amplituden-übermäßigen bzw. freguenzen-unstabilen Atmungseigenschaften des Menschen das Steuersignal TTL eine Abschaltung der auf die Lage des Körperteils fokussierenden Bestrahlungseinrichtung bewirkt. Dies ist besonders wichtig, wenn z.B. der Patient husten würde oder ein zu hohes Herzrhythmus hat, wobei die Planung für die Bestrahlungseinrichtung BE gestört bzw. ungenauer sein könnte.

Der Drucksensor DS bzw. andere Sensorarten zur Erfassung von Über- und Unterdruckzyklen aus dem ein- und ausgehenden Luftstrom müssen nicht unbedingt in der Maske NMM integriert werden, aber könnte am Eingang des Auswertungsmoduls AM angeordnet werden und über einen Luftkanal (Schlauch) mit der Maske NMM in Verbindung stehen. Diese Variante ist z.B. vom Vorteil, wenn die nun magnetischlose bzw. metallfreie Nasen-Mundmaske NMM während eines Abbildungsverfahrens mit Magnetresonanz verwendet wird. Beide Variante (Maske mit oder ohne integriertem Drucksensor) sind vorteilhaft, da derartige Einwegmaske kostengünstig sind und eine sterilisation nicht benötigen.

In Fig. 2 werden Randbedingungen der Bestrahlung in einem A-temzyklus gemäß Fig. 1 skizziert, indem drei mögliche Lage BL des relativ zur Referenzlage RL beweglichen Tumors dargestellt werden. Eine erste und eine zweite Lage (Tumor mit kontinuierlichem bzw. gestrichenem Umfang) mit höchster Entfernung DISTMAX bilden die topographischen Stellen des Tumors bei voller Ein- bzw. Ausatmung des Patienten. Hier zeigt die Figur eine zwei-dimensionale geradlinige Bewegung des Tumors, jedoch kann in der Praxis die Strecke komplexer sein, z.B. in Form eines drei-dimensional gebogenen Volumens.

Nachdem die Referenzlage RL z.B. mittels einer Computertomographie ermittelt bzw. gewählt wurde, wird hier die Energie bei der Bestrahlungsphase über einen als Referenzlage ausgewählten, kreisförmigen Bereich konzentriert. Die Referenzlage RL könnte jedoch variabel sein, z.B. wenn der Strahlgang umlenkbar ist, so dass er die Strecke des Tumors verfolgen kann. Eine dritte Lage BL des Tumors (mit Schraffierungen) wurde innerhalb dieser Referenzlage dargestellt. Gemäß Fig. 1 wird daher bei oder unterhalb einer räumlich tolerierbaren Entfernung TDIST zwischen der beweglichen Lage BL des Körperteils und der Referenzlage RL ein aus dem Auswertungsmodul AM abgegebenes Signal wie das Steuersignal TTL erzeugt, um die auf die Lage des Körperteils fokussierende Bestrahlungseinrichtung BE einzuschalten. Damit wird unter einer vordefinierten Toleranz genauer sichergestellt, dass der gesamte Tumor und nicht seiner Umgebung abgestrahlt wird. In diesen Sinnen auch und oberhalb der räumlich tolerierbaren Entfernung TDIST bewirkt das Steuersignal TTL eine Abschaltung der auf die Lage des Körperteils fokussierenden Bestrahlungseinrichtung BE. Die Referenzlage kann also eine freie Geometrie haben, insbesondere mit Hilfe von einem oder mehreren Strahlengängen, deren Querschnitte mittels steuerbaren Blendenöffnungen veränderbar sind.

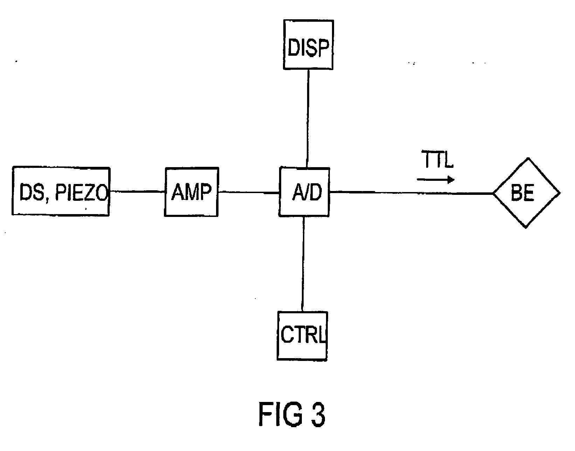

Anschließend wird in Fig. 3 ein Ausführungsbeispiel der erfindungsgemäßen Vorrichtung gemäß Fig. 1 mit möglichen Grundkomponenten dargestellt. Dem Drucksensor DS mit einem Piezo-resistiven Element PIEZO ist ein elektrischer Verstärker AMP für das in der Maske NMM erfasste, elektrisch konvertiere A-temsignal nachgeschaltet. Am Ausgang des Verstärkers AMP ist ein Analog/Digital-Wandler A/D angeschlossen, welcher drei Ausgänge für das Monitor DISP der Elektrorespirographen, für die Bedienungs- bzw. Planungsoberfläche CTRL und für die Steuerung der Bestrahlungseinrichtung BE über das Steuersignal TTL aufweist. Ggf. kann auch die Bestrahlungseinrichtung BE über ein anderes oder weiteres Steuersignal aus der Bedienungs- bzw. Planungsoberfläche CTRL kontrolliert werden.

Der Gegenstand der Erfindung setzt voraus, dass die erfinderische Vorrichtung für mindestens zwei Hauptverwendungen geeignet ist: einerseits für die Erfassung und Datenverarbeitung von Messdaten der räumlichen Bewegung der Lage des Körperteils in Anhängigkeit von Atmungseigenschaften eines Patienten in einer hard- oder/und softwaremässige Planungsoberfläche gespeichert werden; andererseits werden anschließend diese Messdaten aus der Planungsoberfläche für eine Simulation oder/und für eine Steuerung einer ein- und ausschaltbaren Bestrahlungseinrichtung mittels eines geeigneten Therapieplanungssystems verwendet.

Selbstverständlich können ggf. ein oder weitere Mitteln zur "direkten" Visualisierung bzw. zur "indirekten" Verfolgung des Tumors neben seitlich zur erfinderischen Vorrichtung verwendet werden, um die Robustheit bzw. die Reproduzierbarkeit einer Computertomographie oder/und einer dauerhaften Bestrahlungstherapie zu steigern. Beispielsweise kann als ein aktivierbares Visualisierungsmittel des Tumors ein Verfahren zur Ionisierungsstrahlung (schnelle konische Bestrahlung des Thorax bis zu einer Amorph-Siliciumplatte, so dass der Tumor lokalisiert wird) oder eine Computertomographie in zeitlichen Abständen, z.B. gleichzeitig zur tatsächlichen Bestrahlung, verwendet werden.

## Patentansprüche

1. Vorrichtung zur räumlichen Verfolgung einer beweglichen Lage (BL) eines Körperteils im verformbaren Thorax-Bereich eines atmenden Menschen, bei welcher:
- eine Messeinheit (NMM) zur Ermittlung eines vom Kopf des Menschen ein- und ausgehenden Luftstromes mit einem Auswertungsmodul (AM) derart gekoppelt ist, dass ein aus der Messeinheit ausgehendes Meßsignal (MS) ein Maß für zumindest eine im Auswertungsmodul ermittelbare Entfernung (DIST) zwischen der beweglichen Lage des Körperteils und einer Referenzlage (RL) bildet,
- die Messeinheit eine Nasen-Mundmaske (NMM) umfasst, über welche der ein- und ausgehenden Luftstrom als von Mund und Nase summiertes Atemluftstrom ermittelt wird,
- zur Erfassung von Über- und Unterdruckzyklen aus dem ein- und ausgehenden Luftstrom die Messeinheit einen Drucksensor (DS) aufweist,
- die Nasen-Mundmaske (NMM) eine Einwegmaske ist,
**dadurch gekenntzeichnet, dass**
der Drucksensor (DS) ein piezo-resistives Element aufweist, und dass
- der Drucksensor (DS) in der Nasen-Mundmaske (NMM) integriert ist.

2. Vorrichtung nach Anspruch 1,
**dadurch gekennzeichnet, dass**
die Referenzlage (RL) mittels einer Computertomographie oder eines Verfahrens mit Magnetresonanz ermittelt wird.

3. Vorrichtung nach einem der Ansprüche 1 bis 2,
**dadurch gekennzeichnet, dass**
bei oder unterhalb einer räumlich tolerierbaren Entfernung (TDIST) zwischen der beweglichen Lage (BL) des Körperteils und der Referenzlage (RL) ein aus dem Auswertungsmodul abgegebenes Steuersignal (TTL) erzeugt wird, um eine auf die Lage des Körperteils fokussierende Bestrahlungseinrichtung (BE) einzuschalten.

4. Vorrichtung nach Anspruch 3,
**dadurch gekennzeichnet, dass**
oberhalb der räumlich tolerierbaren Entfernung (TDIST) das Steuersignal (TTL) eine Abschaltung der auf die Lage des Körperteils fokussierenden Bestrahlungseinrichtung (BE) bewirkt.

5. Vorrichtung nach Anspruch 3 oder 4,
**dadurch gekennzeichnet, dass**
ein weiteres, aus dem Auswertungsmodul abgegebenes Steuersignal (MOV) ein Positionierungsmodul des Menschenkörpers und/oder der Bestrahlungseinrichtung (BE) steuert, so dass räumliche Abweichungen zwischen der beweglichen Lage des Körperteils und der Referenzlage minimiert werden.

6. Vorrichtung nach einem der Ansprüche 3 bis 5,
**dadurch gekennzeichnet, dass**
bei unerwarteten, wie amplituden-übermäßigen bzw, frequenzenunstabilen Atmungseigenschaften des Menschen das Steuersignal (TTL) eine Abschaltung der auf die Lage des Körperteils fokussierenden Bestrahlungseinrichtung bewirkt.

7. Vorrichtung nach einem der voranstehenden Ansprüche,
**dadurch gekennzeichnet, dass**
mittels einer Kalibrierung zwischen Atmungseigenschaften des Menschen und gemessen Positionen oder Bewegungen der Lage des Körperteils das Auswertungsmodul Maßstäbe für die Entfernung (DIST) und ggf. drei-dimensionale Koordinaten der beweglichen Lage (BL) des Körperteils in einem zur Referenzlage (RL) bezogenen Koordinatensystem ermittelt.

8. Vorrichtung nach einem der voranstehenden Ansprüche,
**dadurch gekennzeichnet, dass**
das aus der Messeinheit ausgehendes Meßsignal (MS) an einem Monitor (DISP) in dynamischer Weise visualisierbar ist.

9. Vorrichtung nach einem der voranstehenden Ansprüche,
**dadurch gekennzeichnet, dass**
die bewegliche Lage des Körperteils ein Tumor oder ein Karzinom ist.

10. Vorrichtung nach Anspruch 9,
**dadurch gekennzeichnet, dass**
mindestens ein Visualisierungsmittel des Tumors oder des Karzinoms wie durch ein Verfahren zur Ionisierungsstrahlung oder eine Computertomographie in zeitlichen Abständen aktivierbar ist.

11. Vorrichtung nach einem der voranstehenden Ansprüche,
**dadurch gekennzeichnet, dass**
vorkalibrierte Messdaten der räumlichen Bewegung der Lage (BL) des Körperteils in Anhängigkeit von Atmungseigenschaften eines Patienten in einer hard- oder/und softwaremässige Planungsoberfläche gespeichert werden, derart dass in Verbindung mit einem Therapieplanungssystem eine anschließende Steuerung einer ein- und ausschaltbaren Bestrahlungseinrichtung für den Patient zumindest simulierbar ist.

## Claims

1. Device for spatial tracking of a moving position (BL) of a body part in the deformable thorax region of a breathing human being, wherein:
- a measuring unit (NMM) for determining an air flow entering and exiting the head of the human being is coupled to an evaluation module (AM) in such a way that a measurement signal (MS) emanating from the measuring unit forms a measure for at least one distance (DIST), determinable in the evaluation module, between the moving position of the body part and a reference position (RL),
- the measuring unit comprises a nose-mouth mask (NMM) via which the entering and exiting air flow is determined as an aggregate respiratory air flow from mouth and nose,
- the measuring unit has a pressure sensor (DS) for detecting positive pressure and negative pressure cycles from the entering and exiting air flow,
- the nose-mouth mask (NMM) is a disposable mask,
**characterised in that**
the pressure sensor (DS) has a piezoresistive element and that the pressure sensor (DS) is integrated in the nose-mouth mask (NMM).

2. Device according to claim 1,
**characterised in that**
the reference position (RL) is determined by means of a computed tomography scan or a method using magnetic resonance imaging.

3. Device according to one of claims 1 to 2,
**characterised in that**
a control signal (TTL) emitted from the evaluation module is generated at or below a spatially tolerable distance (TDIST) between the moving position (BL) of the body part and the reference position (RL) in order to switch on an irradiation apparatus (BE) focussing on the position of the body part.

4. Device according to claim 3,
**characterised in that**
above the spatially tolerable distance (TDIST) the control signal (TTL) causes the irradiation apparatus (BE) focussing on the position of the body part to be switched off.

5. Device according to claim 3 or 4,
**characterised in that**
a further control signal (MOV) emitted from the evaluation module controls a positioning module of the human body and/or the irradiation apparatus (BE) so that spatial deviations between the moving position of the body part and the reference position are minimised.

6. Device according to one of claims 3 to 5,
**characterised in that**
the control signal (TTL) causes the irradiation apparatus focussing on the position of the body part to be switched off in the event of unexpected, such as excessive amplitude or frequency unstable, respiratory characteristics of the human being.

7. Device according to one of the preceding claims,
**characterised in that**
by means of a calibration between respiratory characteristics of the human being and measured positions or movements of the position of the body part, the evaluation module determines measures for the distance (DIST) and where applicable three-dimensional coordinates of the moving position (BL) of the body part in a coordinate system referred to the reference position (RL).

8. Device according to one of the preceding claims,
**characterised in that**
the measurement signal (MS) emanating from the measuring unit can be visualised in a dynamic manner on a monitor (DISP).

9. Device according to one of the preceding claims,
**characterised in that**
the moving position of the body part is a tumour or a carcinoma.

10. Device according to claim 9,
**characterised in that**
at least one visualisation means for visualising the tumour or the carcinoma such as by way of an ionising radiation method or a computed tomography scan can be activated at time intervals.

11. Device according to one of the preceding claims,
**characterised in that**
pre-calibrated measurement data of the spatial movement of the position (BL) of the body part as a function of respiratory characteristics of a patient is stored in a hardware- or software-based planning interface in such a way that a subsequent control of an irradiation apparatus which can be switched on and off can at least be simulated for the patient in conjunction with a therapy planning system.

## Revendications

1. Dispositif pour suivre dans l'espace une position (BL) mobile d'une partie du corps de la région thoracique déformable d'une personne qui respire, dans lequel :
- une unité (NMM) de mesure, pour déterminer un courant d'air entrant et sortant de la tête de la personne, est couplée à un module (AM) d'exploitation de façon à ce qu'un signal (MS) de mesure sortant de l'unité de mesure forme une mesure d'au moins un éloignement (DIST), pouvant être déterminé dans le module d'exploitation, entre la position mobile de la partie du corps et une position (RL) de référence,
- l'unité de mesure comprend un masque (NMM) pour le nez et pour la bouche, par lequel le courant d'air entrant et sortant est déterminé sous la forme d'un courant d'air de respiration cumulé par la bouche et le nez,
- pour déterminer des cycles de surpression et de dépression à partir du courant d'air entrant et sortant, l'unité de mesure a un capteur (DS) de pression,
- le masque (NMM) pour le nez et la bouche est un masque à jeter,
**caractérisé en ce que** le capteur (DS) de pression a un élément piézorésistif et **en ce que** le capteur (DS) de pression est intégré au masque (NMM) pour le nez et la bouche.

2. Dispositif suivant la revendication 1,
**caractérisé en ce que**
la position (RL) de référence est déterminée au moyen d'une tomographie par ordinateur ou d'un procédé par résonance magnétique.

3. Dispositif suivant l'une des revendications 1 à 2,
**caractérisé en ce que**
à ou en dessous d'un éloignement (TDIST) dans l'espace qui peut être toléré, entre la position (BL) mobile de la partie du corps et la position (RL) de référence, il est produit un signal (TTL) de commande, émis par le module d'exploitation, pour brancher un dispositif (BE) d'envoi d'un rayonnement focalisé sur la position de la partie du corps.

4. Dispositif suivant la revendication 3,
**caractérisé en ce**
**qu'**au-dessus de l'éloignement (TDIST) dans l'espace qui peut être toléré, le signal (TTL) de commande provoque un arrêt du dispositif (BE) d'envoi d'un rayonnement focalisé sur la position de la partie du corps.

5. Dispositif suivant la revendication 3 ou 4,
**caractérisé en ce**
**qu'**un autre signal (MOV) de commande, émis par le module d'exploitation, commande un module de mise en position du corps de la personne et/ou du dispositif (BE) d'envoi d'un rayonnement de manière à minimiser des écarts dans l'espace entre la position mobile de la partie du corps et la position de référence.

6. Dispositif suivant l'une des revendications 3 à 5,
**caractérisé en ce que**
s'il se produit des propriétés de respiration de la personne inattendues, comme des excès d'amplitude ou des instabilités de fréquence, le signal (TTL) de commande provoque un arrêt du dispositif d'envoi d'un rayonnement focalisé sur la position de la partie du corps.

7. Dispositif suivant l'une des revendications précédentes,
**caractérisé en ce**
**qu'**au moyen d'un étalonnage entre des propriétés de respiration de la personne et des positionnements ou des mouvements mesurés de la position de la partie du corps, le module d'exploitation détermine des échelles pour l'éloignement (DIST) et le cas échéant, des coordonnées en trois dimensions de la position (BL) mobile de la partie du corps dans un système de coordonnées rapportées à la position (RL) de référence.

8. Dispositif suivant l'une des revendications précédentes,
**caractérisé en ce que**
le signal (MS) de mesure sortant de l'unité de mesure peut être visualisé de façon dynamique sur un moniteur (DISP).

9. Dispositif suivant l'une des revendications précédentes,
**caractérisé en ce que**
la position mobile de la partie du corps est une tumeur ou un carcinome.

10. Dispositif suivant la revendication 9,
**caractérisé en ce**
**qu'**au moins un moyen de visualisation de la tumeur ou du carcinome, comme par un procédé pour un rayonnement ionisant ou une tomographie par ordinateur, peut être activé à des intervalles de temps.

11. Dispositif suivant l'une des revendications précédentes,
**caractérisé en ce que**
des données de mesure préétalonnées du déplacement dans l'espace de la position (BL) de la partie du corps en fonction des propriétés de respiration d'un patient sont mémorisées dans une surface de planification matérielle ou/logicielle de façon à pouvoir au moins simuler, en liaison avec un système de planification de thérapie, une commande ultérieure d'un dispositif d'exposition du patient à un rayonnement, pouvant être branché et débranché
